# FASCICULE DE BREVET EUROPEEN

(11) **EP 0 967 963 B1**
(45) Date de publication et mention de la délivrance du brevet: **01.03.2006**
(21) Numéro de dépôt: 98913822.7
(22) Date de dépôt: 04.03.1998
(51) Int. Cl.: A61Q 5/04, A61Q 5/06, A61Q 5/08, A61Q 5/10, A61Q 17/04, A61K 8/19, A61K 8/46, A61K 8/55, A61K 8/81, A61K 8/84, A61K 8/892

(54) **PROCEDE DE TRAITEMENT DES CHEVEUX**
HAARBEHANDLUNGSVERFAHREN
HAIR TREATMENT METHOD

(30) Priorité: 04.03.1997 FR 9702537
(43) Date de publication de la demande: 05.01.2000
(73) Titulaire: L'OREAL, 75008 Paris (FR)
(72) Inventeur: MALLE, Gérard, F-77580 Villiers sur Morin (FR); LEROY, Frédéric, F-92210 Saint-Cloud (FR); DUVAULT, Yolanda, F-93600 Aulnay-sous-Bois (FR)
(74) Mandataire: Tanty, François
(86) Numéro de dépôt international: PCT/FR1998/000420
(87) Numéro de publication internationale: WO 1998/038974

(56) Documents cités:
- EP-A- 0 331 750
- WO-A-96/03966
- US-A- 3 396 736
- US-A- 4 041 150

## Description

La présente invention a pour objet un nouveau procédé de traitement des cheveux en vue de leur conférer de nouvelles propriétés appropriées.

Il est bien connu de l'état de la technique que les qualités cosmétiques des cheveux peuvent être améliorées par application de diverses compositions à base d'un ou plusieurs composés actifs permettant de leur conférer diverses propriétés telles que par exemple de la brillance, une facilité de démêlage, du gonflant, de la tenue, de la souplesse, de la nervosité ou encore de la douceur.

Pour obtenir une bonne efficacité, il convient bien entendu que les composés actifs de ces compositions présentent vis-à-vis des fibres kératiniques des cheveux, une certaine affinité ainsi qu'une bonne rémanence.

En d'autres termes, ces composés actifs doivent, dans la mesure du possible, rester fixés sur les cheveux en une quantité suffisante en vue de leur conférer les propriétés recherchées.

Toutefois, dans la mesure où ces principes actifs ne sont pas fixés de façon irréversible mais uniquement par des phénomènes d'adsorption, une élimination progressive par désorption se produit lors des lavages successifs à l'aide de shampooings.

En vue d'améliorer les effets de rémanence, les études ont essentiellement porté sur les traitements tendant à faire pénétrer dans les fibres une proportion importante des principes actifs, soit par une sélection de ces principes actifs ayant une affinité particulière pour les fibres, soit en modifiant les fibres pour accroître leur porosité et favoriser la pénétration.

Il est ainsi connu, pour améliorer la coloration des fibres kératiniques des cheveux, de réaliser simultanément une coloration avec une permanente. En effet, la réduction des liaisons disulfures de la kératine en profondeur permet d'accroître la pénétration du colorant et donc d'obtenir une certaine durabilité de la coloration.

Ce type de traitement n'est toutefois pas sans présenter de sérieux inconvénients car il provoque une dégradation importante, non seulement de l'état de surface des fibres kératiniques, mais également de leurs propriétés mécaniques intrinsèques.

Après d'importantes recherches dans ce domaine, en vue de remédier aux inconvénients rencontrés jusqu'ici, il s'est avéré, de façon surprenante et inattendue, que d'excellents résultats pouvaient être obtenus dans la fixation de composés actifs sur les fibres kératiniques des cheveux sans que ceux-ci subissent de dégradation préjudiciable. Ceci a pu être réalisé en limitant la formation de sites réactifs uniquement à la surface des fibres kératiniques des cheveux à l'aide d'un agent réducteur utilisé dans des conditions et proportions telles que seuls des sites réactifs soient générés à la périphérie de la surface des fibres kératiniques.

On a en effet constaté que la création de sites réactifs uniquement en surface était suffisante, et que ceux-ci étaient remarquablement réactifs pour conduire à une bonne fixation par liaisons covalentes de divers composés actifs, sans que les cheveux ne soient sensiblement modifiés quant à leurs propriétés mécaniques originelles.

La présente invention a donc pour objet un nouveau procédé de traitement des fibres kératiniques des cheveux en vue de leur conférer de nouvelles propriétés appropriées, ce procédé comprenant les étapes consistant à réduire les liaisons disulfures de la kératine en vue de générer, uniquement en surface, à une profondeur inférieure à 10 µm, des sites réactifs et à fixer de façon covalente sur lesdits sites réactifs au moins un composé actif susceptible de conférer de nouvelles propriétés appropriées aux fibres kératiniques des cheveux, ledit composé actif comportant au moins une fonction réactive capable de réagir avec lesdits sites formés à la surface des fibres kératiniques.

Selon l'invention, le procédé de traitement peut être réalisé soit en deux étapes distinctes, à savoir, dans un premier temps la réduction des liaisons disulfures de la kératine, et dans un deuxième temps la fixation du composé actif par liaisons covalentes soit en une seule étape consistant à réaliser simultanément la réduction des liaisons disulfures de la kératine et la fixation du composé actif.

Selon un autre aspect du procédé de traitement, selon l'invention on peut également envisager d'appliquer d'abord sur les fibres kératiniques des cheveux le composé actif et de procéder ensuite à la réduction des liaisons disulfures de la kératine.

La caractéristique essentielle du procédé selon l'invention est de former uniquement à la surface des fibres kératiniques des cheveux, des sites réactifs à l'aide d'une composition réductrice.

Divers agents réducteurs conventionnels peuvent être employés à cet effet, mais leur nature et concentration ainsi que leur méthode d'application devront être telles que seuls des sites réactifs soient uniquement générés en surface seulement du substrat kératinique.

En d'autres termes, la composition réductrice ne devra pas réagir au-delà d'une profondeur de 10 µm, de préférence au-delà d'une profondeur moyenne de 4 à 5 µm, ce qui correspond environ à sa cuticule.

Il s'agit là bien entendu de valeurs moyennes qui pourront varier en fonction du type de traitement et pourront donc être sensiblement inférieures soit supérieures à celles indiquées.

Les sites réactifs générés à là surface des fibres kératiniques sont du type nucléophile et essentiellement des fonctions thiols.

Selon l'invention, la réduction doit être telle qu'elle génère entre 0,1 et 5 % en poids de cystéine par rapport aux acides aminés totaux des fibres kératiniques et de préférence entre 0,1 et 2 % en poids.

Comme agent réducteur de la kératine des cheveux les composés suivants sont employés:
- les thiols tels que l'acide thioglycolique, l'acide thiolactique, l'acide 3-mercaptopropionique, l'acide thiomalique, l'acide 2,3-dimercaptosuccinique, la cystéine, la N-glycyl-L-cystéine, la L-cystéinylglycine ainsi que leurs esters et sels, le thioglycérol, la cystéamine et ses dérivés acylés en C₁-C₄, la N-mésylcystéamine, la N-acétylcystéine, les N-mercaptoalkylamides de sucres tels que le N-(mercapto-2-éthyl) gluconamide, la pantéthéine, les N-(mercaptoalkyl)- ω-hydroxy alkylamides décrits dans la demande de brevet EP-A-354 835, les N-mono ou N,N-dialkylmercapto-4 butyramides décrits dans la demande de brevet EP-A-368 763, les aminomercaptoalkylamides décrits dans la demande de brevet EP-A-432 000, les dérivés des acides N-(mercaptoalkyl)succinamiques et des N-(mercaptoalkyl)succinimides décrits dans la demande de brevet EP-A-465 342, les alkylamino mercaptoalkylamides décrits dans la demande de brevet EP-A-514 282, le mélange azéotrope de thioglycolate de 2-hydroxypropyle et de thioglycolate de (2-hydroxy-1-méthyl)éthyle décrit dans la demande de brevet FR-A-2 679 448, les mercaptoalkylaminoamides décrits dans la demande de brevet FR-A-2 692 481, les N-mercapto alkyl alcane diamides décrits dans la demande de brevet EP-A-653 202 ; Les thiols étant neutralisé à l'aide d'un polyhydroxyde d'ammonium quaternaire ;
- les dérivés du phosphore tels que les phosphines
ou les phosphites ;
- les polymères hyperbranchés et les dendrimères porteurs de fonctions terminales thiols, tels que ceux décrits dans la demande de brevet FR 97 04085 et répondant à la formule (I) : 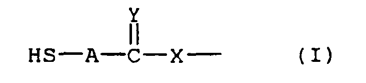
dans laquelle :
Y représente l'atome d'oxygène ou un groupement NH,
A représente un groupement alcane di-yle en C₁₋C₁₂, linéaire, ramifié ou cyclique, saturé ou insaturé ;
ce groupement alcane di-yle peut être éventuellement interrompu par un ou plusieurs hétéroatomes, comme O ou N ;
ce groupement alcane di-yle peut éventuellement être substitué par une fonction :
   - amino : -NH₂, éventuellement sous forme d'un sel d'un acide minéral ou organique,
   - acylamino : -NH-COR, dans lequel R représente un groupement alkyle en C₁-C₁₀ linéaire, ramifié ou cyclique, saturé ou insaturé,
   - acide carboxylique,
   - ester en C₁-C₁₀;
X représente un groupement nucléophile.

Selon un mode de réalisation particulièrement préféré du procédé selon l'invention, on utilise en tant qu'agent réducteur une phosphine ou un sel de phosphine d'un acide minéral ou organique.

Parmi les phosphines qui ont conduit à des résultats particulièrement intéressants quant à la formation de sites réactifs en surface des fibres kératiniques des cheveux, on peut mentionner celles de formule : 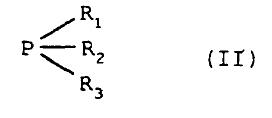
dans laquelle :
R₁, R₂ et R₃, identiques, représentent :
   (a) -(CH₂)ₙ-CH₃
   (b) 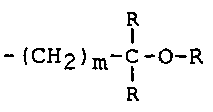
   (c) -(CH₂)ₙ-COOR
   (d) - (CH₂)ₙ-CONRR' et
   (e) -(CH₂)ₙ-NRR'
      n = 1 à 3
      m = 0 ou 1 à 3
      R et R', identiques ou différents, représentent un atome d'hydrogène ou un radical alkyle, linéaire ou ramifiée en C₁-C₄ et les sels desdits composés de formule (II).

Parmi les sels des phosphines de formule (II), on peut notamment citer les chlorhydrates, bromhydrates, sulfates, citrates, oxalates et acétates.

Parmi les phosphines de formule générale (II) particulièrement préférées selon l'invention, on peut notamment citer la tris(2-carboxyéthyl)phosphine ou la tris(hydroxyméthyl)phosphine qui présentent notamment l'avantage d'être non seulement inodores et hydrosolubles mais également stables vis-à-vis de l'oxygène.

Les phosphines de formule générale (II) sont connues et ont été décrites notamment, dans les brevets US-3 754 035 et US-3 489 811 ainsi que dans le EP-339 217 et la publication J.A. Burns et al. J.O.C. 56 2648-2650 (1991).

L'agent réducteur est de préférence utilisé en solution aqueuse dans des conditions telles que sa pénétration soit faible pour limiter la réduction des liaisons disulfures uniquement en surface.

Lorsqu'on utilise un thiol tel que par exemple l'acide thioglycolique, sa concentration est généralement comprise entre 0,05 et 0,5 M, le pH de la solution aqueuse est de préférence compris entre 6,5 et 9, le temps de contact est généralement compris entre 1 et 10 minutes et de préférence entre 2 et 5 minutes, le pH étant ajusté à l'aide d'un polyhydroxyde d'ammonium quaternaire tel que :
(a) les homopolymères comportant comme constituant principal de la chaîne des motifs répondant à la formule (III) : 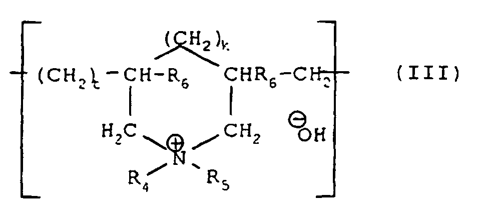
   dans laquelle :
   k et t sont égaux à 0 ou 1, la somme k + t étant égale à 1 ;
   R₄ et R₅ représentent, indépendamment l'un de l'autre, un radical alkyle ayant de 1 à 22 atomes de carbone, un radical hydroxyalkyle dans lequel le groupe alkyle a de préférence de 1 à 5 atomes de carbone, un radical amidoalkyle dans lequel le groupe alkyle a de préférence de 1 à 5 atomes de carbone,
   ou bien R₄ et R₅ peuvent représenter, conjointement avec l'atome d'azote auquel ils sont rattachés, des radicaux hétérocycliques tels que pipéridinyle ou morpholinyle ;
   R₆ représente un atome d'hydrogène ou un radical méthyle ;
(b) les polymères de diammonium quaternaire contenant des motifs récurrents répondant à la formule (IV) : 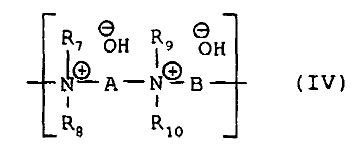
   dans laquelle :
   R₇, R₈, R₉ et R₁₀, identiques ou différents, représentent des radicaux aliphatiques, alicycliques, ou arylaliphatiques contenant de 1 à 20 atomes de carbone ou des radicaux hydroxyaliphatiques inférieurs en C₁-C₅, ou bien R₇, R₈, R₉ et R₁₀, pris ensemble ou séparément, forment avec les atomes d'azote auxquels ils sont rattachés, des hétérocycles contenant éventuellement un second hétéroatome autre que l'azote, ou bien encore R₇, R₈, R₉ et R₁₀, représentent un radical alkyle linéaire ou ramifié en C₁-C₆ substitué par une fonction nitrile, ester, acyle, amide ou -COOR₁₁-Q ou -CO-NH-R₁₁-Q dans lesquels R₁₁ est un alkylène et Q est un groupe ammonium quaternaire ;
   A et B représentent des groupements polyméthyléniques, contenant de 2 à 20 atomes de carbone, linéaires ou ramifiés, saturés ou insaturés, et pouvant contenir, liés à ou bien intercalés dans la chaîne principale, un ou plusieurs cycle(s) aromatique(s), un ou plusieurs atome(s) d'oxygène, de soufre ou des groupements sulfoxyde, hydroxyle, ammonium quaternaire, uréido, amide ou ester ;
   A, R₇ et R₈ peuvent en outre former avec les deux atomes d'azote auxquels ils sont rattachés, un cycle pipérazinique ; par ailleurs, si A représente un radical alkylène ou hydroxyalkylène, linéaire ou ramifié, saturé ou insaturé, B peut également désigner un groupement -(CH₂)ₙ-CO-D-OC-(CH₂)ₙ- dans lequel D représente :
      (i) un reste de glycol de formule -O-Z-O-dans laquelle Z représente un radical hydrocarboné linéaire ou ramifié, ou bien un groupement -(CH₂-CH₂-O)ₓ-CH₂-CH₂- ou -[CH₂-CH-(CH₃)-O]_{y}-CH₂-CH-(CH₃)- dans lesquels x et y représentent un nombre entier de 1 à 4 correspondant à un degré de polymérisation défini et unique, ou un nombre quelconque de 1 à 4 correspondant à un degré de polymérisation moyen,
      (ii) un reste de diamine bis-secondaire tel qu'un dérivé de pipérazine,
      (iii) un reste de diamine bis-primaire de formule -NH-Y-NH- dans laquelle Y représente un radical hydrocarboné linéaire ou ramifié ou bien le radical divalent -CH₂-CH₂-S-S-CH₂-CH₂-,
      (iv) un groupement uréylène de formule -NH-CO-NH- ;
(c) les polymères de polyammonium quaternaires constitués de motifs de formule (V) :

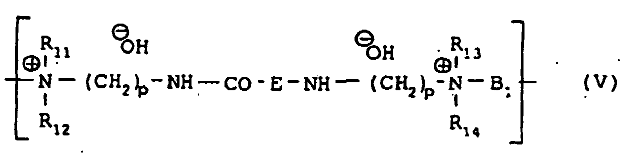

dans laquelle :
R₁₁, R₁₂, R₁₃ et R₁₄, identiques ou différents, représentent un atome d'hydrogène, un radical méthyle, éthyle, propyle, β-hydroxyéthyle, β-hydroxypropyle ou -CH₂-CH₂-(O-CH₂-CH₂)_{q}-OH, q étant un nombre entier de 0 à 6 et R₁₁, R₁₂, R₁₃ et R₁₄ n'étant pas simultanément un atome d'hydrogène,
B₁ représente un groupement polyméthylénique contenant de 2 à 20 atomes de carbone, linéaire ou ramifié, saturé ou insaturé et contenant, liés à ou intercalés dans la chaîne principale, un ou plusieurs cycle(s) aromatique(s) ou un ou plusieurs atome(s) d'oxygène, de soufre ou des groupements sulfoxyde, sulfone, disulfure, amino, alkylamino, hydroxyle, ammonium quaternaire, uréido, amide ou ester,
de préférence B₁ représente un radical de formule

   -CH₂-CH₂-O-CN₂-CH₂-,
p représente un nombre entier variant de 1 à 6 environ,
E peut être nul ou représenter un groupement -(CH₂)ᵣ-CO- dans lequel r représente un nombre égal à 4 ou à 7.

Parmi ces polymères de formules (III), (IV) et (V), on utilise de préférence ceux choisis parmi :
- le composé de formule (IV) dans laquelle R₇, R₈, R₉ et R₁₀ représentent le radical méthyle ; A représente le radical -(CH₂)₃- et B représente le radical -(CH₂)₆-,
- le composé de formule (IV) dans laquelle R₇ et R₈ représentent le radical éthyle, R₉ et R₁₀ représentent le radical méthyle et A et B représentent -(CH₂)₃-,
- le composé de formule (IV) dans laquelle R₇, R₈, R₉ et R₁₀ représentent le radical méthyle et A et B représentent - (CH₂)₃-,
- le composé de formule (IV) dans laquelle R₇, R₈, R₉ et R₁₀ représentent le radical méthyle et A et B représentent -(CH₂)₆-,
- le composé de formule (IV) dans laquelle R₇, R₈, R₉ et R₁₀ représentent le radical méthyle ; A représente le radical -(CH₂)₃- et B représente le radical -(CH₂)₉-,
- le composé de formule (IV) dans laquelle R₇, R₈, R₉ et R₁₀ représentent le radical méthyle ; A représente le radical -(CH₂)₃- et B représente le radical - (CH₂)₂-O- (CH₂)₂-,
- le composé de formule (IV) dans laquelle R₇, R₈, R₉ et R₁₀ représentent le radical méthyle ; A représente le radical -(CH₂)₃- et B représente le radical - (CH₂)₂-O- (CH₂)₂-O- (CH₂)₂-, (IV)
- le composé de formule dans laquelle R₇, R₈, R₉ et R₁₀ représentent le radical méthyle ; A représente le radical -(CH₂)₂-O-(CH₂)₂- et B représente le radical -(CH₂)₂-O-(CH₂)₂-O-(CH₂)₂-,
- le composé de formule (V) dans laquelle R₁₁, R₁₂, R₁₃ et R₁₄ représentent le radical méthyle ; B₁ représente le radical -CH₂-CH₂-O-CH₂-CH₂- et E représente le radical -(CH₂)₄-CO-,
- le composé de formule (V) dans laquelle R₁₁, R₁₂, R₁₃ et R₁₄ représentent le radical méthyle ; B₁ représente le radical -CH₂-CH₂-O-CH₂-CH₂- et E représente le radical (CH₂)₇-CO-,
- le composé de formule (V) dans laquelle R₁₁, R₁₂, R₁₃ et R₁₄ représentent le radical méthyle ; B₁ représente le radical -CH₂-CH₂-O-CH₂-CH₂- et E désigne la valeur zéro,
- le composé de formule (III) dans laquelle t = 1 et k = 0 ; R₄ et R₅ représentent le radical méthyle et R₆ représente un atome d'hydrogène.

Les polyhydroxydes d'ammonium quaternaires des formules (III), (IV) et (V) sont préparés à partir des polyhaloqénures d'ammonium quaternaires correspondants (l'halogénure étant Cl^{⊖} ou Br^{⊖}) selon des méthodes connues en soi qui sont :
- soit un échange avec une résine basique telle que la résine IRA 400, la résine DOWEX 1 x 10, etc. en milieu aqueux,
- soit par précipitation des halogénures sous forme de bromure ou de chlorure d'argent à l'aide d'oxyde d'argent Ag₂O en milieu aqueux.

Lorsqu'on utilise un dérivé du phosphore tel qu'une phosphine, sa concentration est généralement comprise entre 10⁻³ M et 1 M et de préférence entre 10⁻² M et 0,5 M, le temps de contact est généralement compris entre 30 secondes et 1 heure et de préférence entre 1 et 30 minutes, et le pH de la solution aqueuse est de préférence compris entre 3 et 9 et plus particulièrement entre 4 et 7.

Lorsqu'on utilise un polymère hyperbranché ou un dendrimère porteur de fonctions thiols, sa concentration est telle que le titre en thiols de la solution est compris généralement entre 100 et 5000 méq/l, de préférence entre 500 et 2000 méq/l, le temps de contact est généralement compris entre 30 secondes et 1 heure, de préférence entre 1 et 30 minutes, le pH de la solution aqueuse est de préférence compris entre 5 et 11 et plus particulièrement entre 7,5 et 10,5.

L'étape de réduction des liaisons disulfures est généralement réalisée à température ambiante, mais celle-ci peut également être effectuée à une température inférieure à 60°C.

Il va de soi que ces différents paramètres concernant la concentration, le pH, la température et le temps de contact sont interdépendants et qu'il conviendra donc d'en tenir compte. Ainsi, par exemple, une augmentation de la concentration ou une élévation de la température aura pour conséquence une diminution notable du temps de contact.

Lorsque le procédé de traitement selon l'invention est réalisé en deux temps, les fibres kératiniques après réduction des liaisons disulfures de la kératine peuvent être rincées à l'eau avant de fixer le composé actif.

Les composés actifs pouvant être fixés de façon covalente sur les fonctions nucléophiles générées peuvent être de nature très variée et leur choix dépend des propriétés recherchées. Ces composés actifs peuvent être utilisés tels quels s'ils possèdent des fonctions susceptibles de former des liaisons covalentes avec les fonctions nucléophiles des fibres kératiniques des cheveux.

Lorsque les composés actifs que l'on souhaite fixer par liaisons covalentes ne présentent pas de telles fonctions, celles-ci sont alors préalablement introduites sur le composé actif selon les méthodes connues.

Par fonction réactive, on entend un groupe réactif connu qui permet la formation d'une liaison covalente (par réaction avec des fonctions nucléophiles, en l'occurrence des fonctions sulfhydriles -SH) et qui comporte donc un ou plusieurs nucléofuge(s) X ou bien un ou plusieurs carbone(s) ou liaison(s) activée(s). Les nucléofuges habituels sont les groupes :
Cl, Br, F, -OSO₃M, -OSO₂ alkyle, -OSO₂ aryle, -OSO₂N(alkyle)₂, -OR₁, SR₂, -SOR₂, -SO₂R₂, -S^{⊕}R₂R₃, -SCN, -SCOOR₂, -NR₂R₃, N^{⊕} R₂R₃R₄, 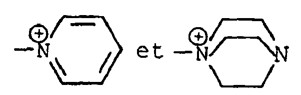
M représentant un atome d'hydrogène, un métal alcalin ou alcalino-terreux ou un reste d'ammonium,
R₁ représente un atome d'hydrogène, un radical alkyle en C₁-C₄, un radical phényle, substitué ou non, le radical PO₃H₂ et ses sels ou le radical acétyle.
R₂, R₃ et R₄, identiques ou différents, représentent un atome d'hydrogène un radical alkyle en C₁-C₄ ou un radical phényle substitué ou non.
Parmi les groupes réactifs les plus connus, on peut notamment citer :
   - les mono et dihalogénotriazines,
   - les dihalogéno quinoxalines, les dihalogéno pyrimidines,
   - les vinylsulfones ou leurs précurseurs β-halogéno ou β-sulfatoéthylsulfones,
   - les acrylates et méthacrylates,
   - les acrylamides et méthacrylamides,
   - les maléimides et halogénomaléimides,
   - les époxydes et dérivés de l'aziridine,
   - les groupes oxazolinium, imidazolium ou thiazolidinium,
   - les halogénures d'acides carboxyliques ou sulfoniques,
   - les esters,
   - les carbamates,
   - les anhydrides,
   - les isothiocyanates et isocyanates,
   - les lactones,
   - les azalactones de structure :

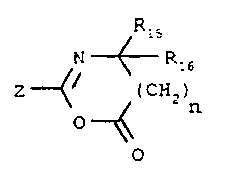

dans laquelle :
Z représente le reste d'un composé actif,
R₁₅ et R₁₆, identiques ou différents, représentent un atome d'hydrogène, un radical alkyle C₁-C₁₂, cycloalkyle C₃-C₁₂, aryle C₅-C₁₂, arényle C₆-C₂₆ comportant de 0 à 3 hétéroatomes choisis parmi S, N et O, ou bien R₁₅ et R₁₆ forment ensemble un carbocycle contenant de 4 à 12 atomes et n est un entier compris entre 0 et 3, etc.

Parmi les composés actifs pouvant être fixés de façon covalente sur les fibres kératiniques des cheveux après réduction des liaisons disulfures de surface, on peut notamment mentionner les agents permettant de les colorer, de les protéger des agressions extérieures (lumière, pollution, eau), de les renforcer, de modifier leur forme, de leur donner du corps, du gonflant, de la légèreté, de la souplesse, de la douceur, de la nervosité, de faciliter le peignage ou le démêlage, de diminuer l'électricité statique ou d'augmenter leur brillance, c'est-à-dire d'une manière générale tout agent permettant de les protéger et/ou d'en améliorer l'aspect et/ou le toucher.

On peut notamment citer, à titre d'exemple et en vue d'illustrer l'invention les composés actifs suivants :
(a) les colorants réactifs destinés à modifier la couleur des cheveux, tels que :
   - les composés de la gamme REMAZOL® commercialisés par la Société DYSTAR,
   - les composés de la gamme PROCION® commercialisés par la Société ZENECA,
   - les composés des gammes CIBACRONE® et LANASOL® commercialisés par la Société CIBA-GEIGY,
   - les composés de la gamme LEVAFIX® commercialisés par la Société BAYER.

   A titre d'exemple, on peut plus particulièrement citer :
   - le Reactive blue 4 (C.I. 61205),
   - le Reactive black 5 (C.I. 20505),
   - le Reactive blue 19 (C.I. 61200),
   - le Reactive orange 16 (C.I. 17757),
   - le Reactive red 4 (C.I. 18105),
   - le Reactive yellow 2 (C.I. 18972),
   - le Reactive yellow 135, et
   - le Reactive red 2 (C.I. 18200).
(b) les filtres solaires réactifs de la famille des dérivés du benzylidènecamphre, des dérivés de l'acide para-aminobenzoïque et de ses esters, des dérivés de l'acide cinnamique et de ses esters, des dérivés salicylés, des dérivés de la benzophénone, des dérivés du dibenzoylméthane, des dérivés du benzotriazole ou du benzimidazole, l'acide anthranilique et ses esters, des dérivés anthranilates ou cyanoacrylates, tels que par exemple ceux de formules suivantes : 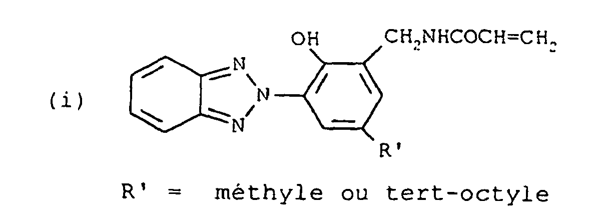 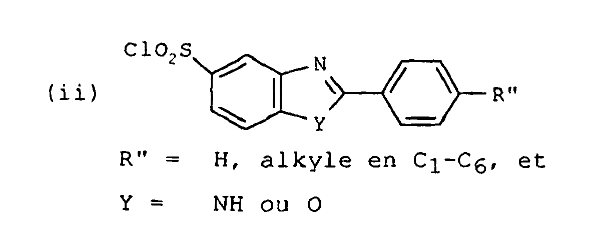 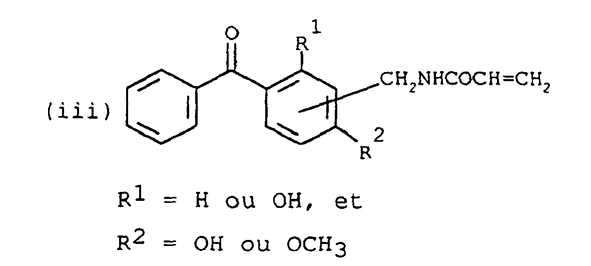 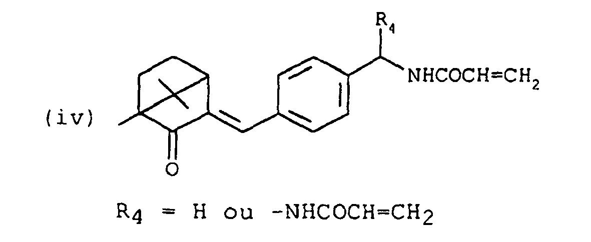 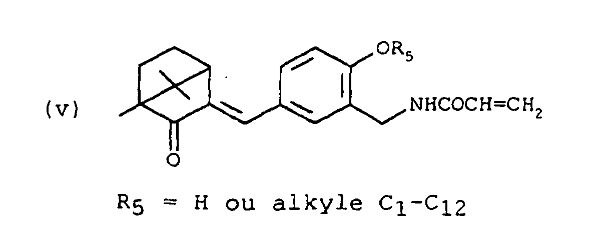 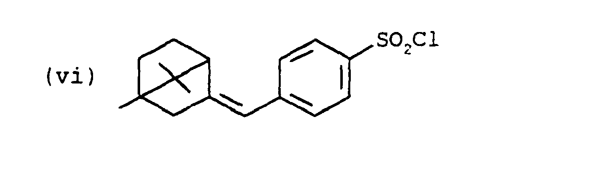
(c) les agents de brillance réactifs, notamment les polymères siliconés tels que les dérivés de polydiméthylsiloxane possédant par exemple des groupes réactifs vinyliques, acryliques ou époxy, etc. On citera à titre d'exemple, les divinypolydiméthylsiloxanes commercialisés par la Société HÜLS-PETRARCH sous les références PS 441, PS 443, PS 445, PS 448 et PS 449.5 ou encore le copolymère vinylméthyl polydiméthylsiloxane commercialisé par la même société sous la référence PS 424 ;
(d) les composés réactifs hydrophobes à chaîne grasse en C₈-C₃₀ tels que par exemple le méthacrylate ou l'acrylate d'octadécyle ou à chaîne perfluorée en C₂ à C₁₈ tels que les FORALKYLS® AC6 et AC8, MAC6 et MAC8 commercialisés par la Société ELF ATOCHEM, l'oxyde d'hexafluoropropène, le perfluorobuten-3-oate de méthyle, etc.

Le composé actif est généralement présent en solution aqueuse à une concentration comprise entre 10⁻³ et 20 % et de préférence entre 10⁻² et 5 % et son pH est généralement compris entre 2 et 10 et de préférence entre 4 et 9.

Le temps de contact nécessaire à la formation des liaisons covalentes est généralement compris entre 1 minute et 1 heure mais de préférence entre 1 et 30 minutes à la température ambiante ou à une température inférieure à 60°C.

La composition contenant l'agent réducteur ainsi que la composition contenant le composé actif peuvent comprendre en outre divers additifs.

Parmi les additifs on peut notamment citer les agents tensio-actifs de type non-ionique, anionique, cationique ou amphotère, les silicones volatiles ou non volatiles, linéaires ou cycliques, les polyorganosiloxanes, les polymères cationiques tels que ceux utilisés dans les compositions des brevets FR-A-2 472 382 et FR-A-2 495 931 et du brevet LU-83703, les amino-acides basiques ou acides, les peptides, les hydrolysats de protéines, les cires, les alcanediols en C₃-C₆, les alcools inférieurs en C₁-C₅, les alcools gras, les acides gras, les alkyléthers d'alkylène, ou de dialkylèneglycol, le glycérol, les agents gélifiants hydrophiles ou lipophiles, les épaississants, les agents de suspension, les agents opacifiants, les agents séquestrants, les colorants, les filtres solaires, les charges, les pigments, les absorbeurs d'odeurs, les agents anti-chute, les agents antipelliculaires, les anti-oxydants, les vitamines, les solvants, les parfums et les conservateurs

Ces différents additifs sont généralement présents en une proportion comprise entre 0,01 et 20 % du poids total de la composition.

Afin d'illustrer l'invention, on va maintenant donner un certain nombre d'exemples du procédé de traitement des cheveux selon l'invention.

### EXEMPLES

### Exemple 1. Procédé de fixation d'un colorant sur des mèches de cheveux

### A. Etape de réduction des mèches de cheveux

Des mèches de cheveux naturels contenant 90 % de cheveux non pigmentés ont été réduites selon les huit méthodes suivantes :

### Méthode 1

Une mèche de cheveux naturels de 1 g a été immergée pendant 5 minutes et à 30°C dans 25 cm³ d'une solution aqueuse 0,5 M de tris(2-carboxyéthyl)phosphine ajustée à pH 9 par de la soude.

### Méthode 2

Une mèche de cheveux naturels de 1 g a été immergée pendant 5 minutes à 30°C dans 5 cm³ d'une solution aqueuse 0,5 M de tris(2-carboxyéthyl)phosphine ajustée à pH 8,5 par de la soude.

### Méthode 3

Une mèche de cheveux naturels de 1 g a été immergée pendant 25 minutes à 30°C dans 5 cm³ d'une solution aqueuse 0,1 M de tris(2-carboxyéthyl)phosphine ajustée à pH 8,5 par de la soude.

### Méthode 4 ne faisant pas partie de l'invention

Une mèche de cheveux naturels de 1 g a été immergée pendant 5 minutes à 30°C dans 25 cm³ d'une solution aqueuse 0,1 M de borohydrure de sodium ajustée à pH 8,5 par de l'acide borique.

### Méthode 5

Une mèche de cheveux décolorés (solubilité alcaline = 20 %) de 1 g a été immergée pendant 5 minutes à 30°C dans 30 cm³ d'une solution aqueuse 0,74 M d'acide thioglycolique ajustée à pH 8 par du dihydroxyde de poly[(diméthyl-iminio)-1,3-propanediyl (diméthyliminio)-1,6-hexanediyle].

### Méthode 6

Une mèche de cheveux naturels de 1 g a été immergée pendant 5 minutes à 30°C dans 30 cm³ d'une solution aqueuse 1 M de L-cystéine, contenant 7,8 g de dihydroxyde de poly[(diméthyliminio)-1,3-propanediyl (diméthyliminio)-1,6-hexa-nediyle], ajustée à pH 9 par de l'ammoniaque.

### Méthode 7

Une mèche de cheveux naturels de 0,1 g a été immergée dans 1 ml d'une solution aqueuse contenant un dendrimère porteur de fonctions thiols dont la préparation est donnée ci-après (titre en thiol : 1340 méq/l) à pH spontané, pendant 30 minutes à 30°C.

### Préparation du dendrimère

A 2 g d'une solution aqueuse à 55,7 g/100 g de dendrimère commercialisé par la Société DENDRITECH sous le nom PAMAM STARBURST à coeur éthylène diamine de génération 1 (8 fonctions NH₂ en surface) dilués par 2 ml d'eau, on ajoute 540 µl de y-thiobutyrolactone (soit 1 équivalent calculé par rapport à l'ensemble des fonctions amines primaires) sous atmosphère inerte à température ambiante. Le milieu, initialement hétérogène, devient rapidement homogène (1 heure). Après 48 heures sous agitation, on ne détecte que des traces de γ-thiobutyrolactone dans le milieu. On lave 3 fois par 10 ml d'éther diéthylique, puis on fait barboter de l'azote dans la phase aqueuse ainsi obtenue pour éliminer toute trace d'éther.

La solution aqueuse ainsi obtenue est analysée par RMN. On constate que toutes les fonctions amines primaires initiales sont sous forme -NH-CO-(CH₂)₃-SH.

La teneur en matière active de cette phase aqueuse est do 37,71 g/100 g.

Masse molaire du produit synthétisé : 2247 g.mol⁻¹

Formule Brute : C₉₄H₁₇₆N₂₀O₂₀S₈

### Méthode 8

Une mèche de cheveux naturels de 0,1 g a été immergée dans 1 ml d'une solution aqueuse contenant un polymère branché polyéthylèneimine porteur de fonctions thiols dont la préparation est donnée ci-après (titre en thiol : 1220 méq/l) à pH spontané, pendant 30 minutes à 30°C.

### Préparation du polymère branché polyéthylèneimine

On dilue 1 g de polyéthylèneimine de poids moléculaire moyen PM=1200 commercialisée par la Société POLYSCIENCES dans 3 g d'eau puis on ajoute à température ambiante 482 µl de γ-thiobutyrolactone (soit 6,75 équivalents molaires calculés par rapport au poids moléculaire moyen du polymère) sous atmosphère inerte à température ambiante. Le milieu, initialement hétérogène, devient rapidement homogène (environ 30 minutes). Après 2 heures sous agitation, on ne détecte plus de γ-thiobutyrolactone dans le milieu. La phase aqueuse donne une réaction positive après révélation par le nitroprussiate de sodium. On constate ainsi que certaines des fonctions amines primaires initiales sont sous forme de -NH-CO-(CH₂)₃-SH.

La teneur en matière active du cette phase aqueuse est de 34,35 g/100 g.

Masse molaire du produit synthétisé : 1889.6 g/mol⁻¹.

Après cette étape de réduction, selon les méthodes 1 à 8, les mèches ont été abondamment rincées à l'eau.

La réduction en surface des liaisons disulfures de la kératine des cheveux selon les méthodes 1 à 8 ci-dessus a pu être mise en évidence à l'aide d'une sonde fluorescente, le 4-(aminosulfonyl)-7-fluoro-2,1,3-benzoxadiazole (ABD-F). Cette molécule réagit en effet sélectivement avec la kératocystéine formée donnant un produit fluorescent. Le traitement des microcoupes transversales du cheveu par une solution de sonde permet ainsi de localiser les zones du cheveu réduites.

A cet effet, les cheveux réduits selon les méthodes 1 à 8 sont inclus dans une résine époxy à durcissement rapide. A partir des différentes inclusions ainsi réalisées, on obtient des coupes transversales de 10 µm à l'aide d'un microtome.

On applique alors sur les coupes quelques gouttes de la solution suivante : 2,16 mg de ABD-F dans 100 ml d'une solution tampon borate pH 8 contenant 576,8 mg de lauryl sulfate de sodium, 37,2 mg d'EDTA et 945,5 mg de borax.

On laisse agir 30 secondes et l'on retire la solution à l'aide d'un papier absorbant. Les coupes sont alors rincées trois fois à l'eau permutée puis séchées et incluses dans du baume du Canada.

L'observation de la fluorescente s'effectue à l'aide d'un microscope optique "Diaplan" équipé avec un bloc de filtres qui permet l'excitation à une longueur d'onde λ de 340-380 nm et l'observation dans des domaines λ > 430. On détermine ainsi aisément que la réduction s'est effectuée en surface à une profondeur superficielle, et non à l'intérieur des fibres de la kératine des cheveux.

### B. Etape de greffage du colorant

On prépare une solution aqueuse 10⁻² M du colorant "Reactive orange 16" (C.I. 17757) et ajuste le pH à 9 par de la soude. On immerge alors dans cette solution la mèche de cheveux réduite selon la Méthode 1 ci-dessus pendant 30 minutes et à 30°C. Puis on répète l'opération avec les autres mèches réduites selon les Méthodes 2 à 8.

On rince ensuite abondamment à l'eau les mèches de cheveux puis on les lave à plusieurs reprises par une solution aqueuse à 10 % de lauryléthersulfate de sodium et on les sèche.

On obtient ainsi des mèches présentant une coloration orangée intense et on constate que le lavage prolongé et la répétition des shampooings ne modifient pas de façon perceptible l'intensité de la coloration par rapport à celle initialement observée.

### C. Etude comparative

Une mèche de cheveux naturels de 1 g a été immergée pendant 30 minutes à 30°C dans 25 ml d'une solution aqueuse 10⁻² M du colorant "Reactive orange 16" (C.I. 17757) dont le pH a été ajusté à 9 par de la soude. La mèche est ensuite abondamment rincée à l'eau puis lavée à plusieurs reprises par une solution aqueuse à 10 % de lauryléther sulfate de sodium et séchée.

Cette mèche n'ayant pas subi d'étape de réduction préalable présente une très faible coloration à peine perceptible à l'oeil et il en est de même, mais dans une moindre mesure, lorsque l'on augmente la température et le temps de contact.

### Exemple 2. Procédé de fixation d'une chaîne grasse sur des mèches de cheveux

On immerge une mèche de cheveux naturels de 1 g préalablement réduite selon la Méthode 1 de l'exemple 1, dans 25 cm³ d'une solution 0,5 x 10⁻² M de méthacrylate d'octadécyle dans un mélange éthanol-eau (9/1) et on ajuste le pH à 9 par de la soude. Après 30 minutes à 45°C la mèche est abondamment rincée à l'éthanol puis à l'eau permutée et séchée.

Les études effectuées sur la mèche montrent qu'elle présente un caractère hydrophobe particulièrement marqué.

### Etude comparative

Une mèche de cheveux naturels de 1 g a été immergée pendant 30 minutes à 45°C dans 25 cm³ d'une solution 0,5 x 10⁻² M de méthacrylate d'octadécyle dans un mélange éthanol-eau (9/1) ajusté à pH 9 par de la soude.

Après traitement la mèche est abondamment rincée à l'éthanol puis à l'eau permutée et séchée.

L'étude de cette mèche n'ayant pas subi d'étape de réduction préalable montre, par des mesures de mouillabilité, qu'elle présente un fort caractère hydrophile. Ceci indique qu'il n'y a eu aucune fixation notable de la chaîne grasse.

### Exemple 3. Procédé de fixation d'un polymère siliconé sur des mèches de cheveux

On immerge une mèche de cheveux naturels de 1 g préalablement réduite selon la Méthode 1 de l'exemple 1, dans 25 cm³ d'une solution à 1 % de divinylpolydiméthylsiloxane, vendu sous la dénomination de "PS 441®" par la Société HÜLS-PETRARCH, dans un mélange éthanol-eau (1/1) et on ajuste le pH à 8 par de la soude. Après 30 minutes à 40°C la mèche est abondamment rincée à l'eau puis lavée à plusieurs reprises par une solution à 10 % de lauryléther sulfate de sodium et séchée.

A l'aide des mesures de mouillabilité, on constate une diminution importante du caractère hydrophile de la mèche ainsi qu'un toucher beaucoup plus agréable et un gain de brillance.

### Exemple 4. Procédé de fixation d'un filtre solaire sur des mèches de cheveux

A. On immerge 5 mèches de 1 g de cheveux bruns naturels préalablement réduites selon la Méthode 2 de l'exemple 1, dans 50 cm³ d'une solution éthanolique 0,1 M de [3-benzo-triazol-2-yl-2-hydroxy-5-(1,1,3,3-tétraméthylbutyl)-benzyl]-acrylamide chauffée à 70°C. On ajoute 20 gouttes de soude 0,1 M et maintient la température pendant 30 minutes.
   Après traitement, les mèches sont rincées 5 fois par 150 cm³ d'éthanol puis 5 fois par 250 cm³ d'eau permutée et séchées.
B. On immerge 2 mèches de 1 g de cheveux bruns naturels préalablement réduites selon la Méthode 3 de l'exemple 1, dans 20 cm³ d'une solution éthanolique 0,1 M de [3-benzo-triazol-2-yl-2-hydroxy-5-(1,1,3,3-tétraméthylbutyl)-benzyl]-acrylamide chauffée à 70°C. On ajoute 8 gouttes de soude 0,1 M et maintient la température pendant 30 minutes.
   Après traitement, les mèches sont rincées 5 fois par 150 cm³ d'éthanol puis 5 fois par 250 cm³ d'eau permutée et séchées.

### C. Etude comparative

Les mèches obtenues en A et B ci-dessus ainsi que 5 mèches de cheveux bruns naturels de référence ont été exposées à la lumière artificielle, dans un simulateur XENOTEST 150S pendant 240 heures. Pendant cette exposition, les cheveux sont aspergés d'eau pendant 5 minutes toutes les 15 minutes.

L'examen des mèches, après cette exposition, a permis de montrer que les mèches obtenues en A et B, par fixation d'un filtre solaire, présentaient une très faible dégradation de leur couleur par rapport aux mèches de référence. L'effet de protection est manifeste après 48 heures d'exposition et est très fortement accentué après 240 heures d'exposition.

### Exemple 5. Effet protecteur des filtres solaires fixés sur des mèches de cheveux teints

On immerge 5 mèches de 1 g de cheveux 90 % blancs naturels, préalablement réduits selon la Méthode 2 de l'exemple 1, dans 50 cm³ d'une solution éthanolique 0,1 M de [3-benzo-triazol-2-yl-2-hydroxy-5-(1,1,3,3-tétraméthyl-butyl)-benzyl]-acrylamide chauffée à 70°C. On ajoute 20 gouttes de soude 0,1 M et maintient la température pendant 30 minutes.

Après traitement, les mèches sont rincées 5 fois par 150 cm³ d'éthanol puis 5 fois par 250 cm³ d'eau permutée et séchées.

Les mèches sont ensuite teintes à l'aide d'un mélange de 20 g de composition colorante "MAJIREL 6.1" et de 30 g d'oxydant crème à 20 volumes d'eau oxygénée. Après 30 minutes de pause, les mèches sont rincées à l'eau puis lavées au shampooing.

Le même traitement a également été effectué sur 5 mèches ayant été préalablement réduites selon la Méthode 3 de l'exemple 1.

Après exposition au Xenotest dans les mêmes conditions qu'à l'exemple 4, on n'a noté qu'une très faible dégradation de la couleur des mèches par rapport à des mèches témoins, c'est-à-dire uniquement teintes à l'aide du mélange colorant ci-dessus.

### Exemple 6. Procédé permettant de déjaunir les cheveux blancs

### A. Etape de réduction des cheveux

Après humidification de cheveux gris à 90 % de blancs, naturels ou permanentés, on a appliqué, pendant 5 minutes à température ambiante, à raison de 10 g pour 3 g de cheveux, la lotion suivante :
- Tris(2-carboxyéthyl)phosphine .........14,33 g
- Hydroxyéthylcellulose ..................1,00 g
- Ammoniaque à 20 % de NH₃ q.s.p. pH 6
- Eau déminéralisée q.s.p..............100,00 g

Les cheveux ont ensuite été rincés à l'eau et essorés.

### B. Etape de greffage de la composition déjaunissante

10 g de la composition déjaunissante suivante ont été appliqués pendant 5 minutes, puis les cheveux ont été rincés, lavés à l'aide d'un shampooing standard puis séchés.

On a constaté que le jaunissement des cheveux blancs était totalement estompé, tout en gardant un reflet naturel.

### Composition déjaunissante :

- Procion YELLOW MX-8G® commercialisé par la Société ZENECA ..........0,01 g
- Procion RED MX-5B® commercialisé par la Société ZENECA 0,01 g
- Lanasol bleu 3 G commercialisé par la Société CIBA-GEIGY .............. 0,01 g
- Hydroxyéthylcellulose .................. 1,00 g
- Acide lactique q.s.p. pH 4
- Eau déminéralisée q.s.p. ............. 100,00 g

## Revendications

1. Procédé de traitement des fibres kératiniques des cheveux en vue de leur conférer de nouvelles propriétés appropriées, **caractérisé par le fait qu'**il comprend les étapes consistant à réduire les liaisons disulfures de la kératine des cheveux en vue de générer, uniquement en surface desdites fibres à une profondeur inférieure à 10 µm, des sites réactifs, la réduction des liaisons disulfures étant réalisée à l'aide d'un agent réducteur choisi parmi les thiols neutralisés à l'aide d'un polyhydroxyde d'ammonium quaternaire, les phosphines et les phosphites, les polymères hyperbranchés et les dendrimères porteurs de fonctions terminales thiols, et à fixer de façon covalente sur lesdits sites réactifs au moins un composé actif susceptible de conférer de nouvelles propriétés appropriées aux fibres kératiniques des cheveux, ledit composé actif comportant au moins une fonction réactive capable de réagir avec lesdits sites réactifs formés en surface des fibres kératiniques.

2. Procédé selon la revendication 1, **caractérisé par le fait que** l'on procède dans un premier temps à la réduction des liaisons disulfures de la kératine et qu'après avoir éventuellement effectué un rinçage à l'eau, on procède, dans un deuxième temps, à la fixation du composé actif.

3. Procédé selon la revendication 1, **caractérisé par le fait que** l'on procède simultanément à la réduction des liaisons disulfures de la kératine et à la fixation du composé actif.

4. Procédé selon l'une quelconque des revendications précédentes, **caractérisé par le fait que** la réduction est réalisée à une profondeur d'environ 4 à 5 µm.

5. Procédé selon l'une quelconque des revendications précédentes, **caractérisé par le fait que** la réduction est réalisée en vue de générer entre 0,1 et 5 % en poids de cystéine par rapport aux acides aminés totaux des fibres kératiniques des cheveux.

6. Procédé selon la revendication 5, **caractérisé par le fait que** la réduction est réalisée en vue de générer entre 0,1 et 2 % en poids de cystéine par rapport aux acides aminés totaux des fibres kératiniques des cheveux.

7. Procédé selon l'une quelconque des revendications précédentes, **caractérisé par le fait que** l'agent réducteur est une phosphine de formule générale: 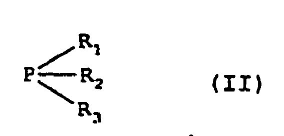
dans laquelle :
R₁, R₂ et R₃, identiques; représentent :
(a) -(CH₂)ₙ-CH₃
(b) 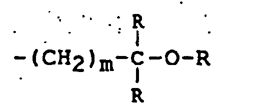
(c) -(CH₂)ₙ-COOR
(d) -(CH₂)ₙ-CONRR' et
(e) -(CH₂)ₙ-NRR'
n = 1 à 3
m = 0 ou 1 à 3
R et R', identiques ou différents représentent un atome d'hydrogène ou un radical alkyle, linéaire ou ramifié, en C₁-C₄ et les sels d'un acide minéral ou organique desdits composés de formule (II).

8. Procédé selon la revendication 7, **caractérisé par le fait que** les sels des phosphines de formule (II) sont choisis parmi les chlorhydrates, bromhydrates, sulfates, citrates, oxalates et acétates.

9. Procédé selon l'une quelconque des revendications 7 à 8, **caractérisé par le fait que** la phosphine est choisie parmi la tris(2-carboxyéthyl)phosphine et la tris(hydroxy-méthyl)phosphine.

10. Procédé selon l'une quelconque des revendications 7 à 9, **caractérisé par le fait que** la phosphine est présente à une concentration comprise entre 10⁻³ M et 1 M.

11. Procédé selon l'une quelconque des revendications précédentes, **caractérisé par le fait que** le pH de la composition de l'agent réducteur est compris entre 3 et 9 et de préférence entre 4 et 7.

12. Procédé selon l'une quelconque des revendications précédentes, **caractérisé par le fait que** le temps de contact de la solution aqueuse de l'agent réducteur avec les fibres kératiniques est compris entre environ 30 secondes et 1 heure, la température étant comprise entre la température ambiante et une température inférieure à 60°C.

13. Procédé selon l'une quelconque des revendications précédentes, **caractérisé par le fait que** le composé actif est choisi parmi les colorants, les filtres solaires, les agents de brillance, et les composés hydrophobes, ledit composé actif étant porteur d'au moins une fonction nucléofuge.

14. Procédé selon l'une quelconque des revendications précédentes, **caractérisé par le fait que** le composé actif est utilisé en solution aqueuse à une concentration comprise entre environ 10⁻³ % et 20 %, ladite solution ayant un pH compris entre environ 2 et 10.

15. Procédé selon l'une quelconque des revendications précédentes, **caractérisé par le fait que** le temps de contact de la solution aqueuse du composé actif est généralement compris entre environ 1 minute et 1 heure, la température étant comprise entre la température ambiante et une température inférieure à 60°C.

## Patentansprüche

1. Verfahren zur Behandlung der Keratinfasern der Haare, um diesen neue angemessene Eigenschaften zu verleihen, **dadurch gekennzeichnet, dass** es die Schritte umfasst, bestehend aus dem Reduzieren der Disulfidbindungen des Haarkeratins, um nur an der Oberfläche dieser Fasern bis zu einer Tiefe von 10 µm reaktive Stellen zu erzeugen, wobei die Reduktion der Disulfidbindungen mit Hilfe eines Reduktionsmittels ausgeführt wird, ausgewählt unter den mit Hilfe eines quatemären Ammoniumpolyhydroxids neutralisierten Thiolen, den Phosphinen und den Phosphiten, sowie den hyperverzweigten und dendrimeren Polymeren, welche terminale Thiolfunktionen tragen, und dem Fixieren mindestens einer aktiven Verbindung auf den reaktiven Stellen auf kovalente Weise, welche Verbindung die neuen angemessenen Eigenschaften, an die Keratinfasern des Haars verleihen kann, wobei die aktive Verbindung mindestens eine reaktive Funktion trägt, die mit den reaktiven Stellen reagieren kann, die auf der Oberfläche der Keratinfasern gebildet sind.

2. Verfahren nach Anspruch 1, **dadurch gekennzeichnet, dass** man in einem ersten Schritt mit der Reduktion der Disulfidbindungen des Keratins vorgeht und, nachdem man gegebenenfalls eine Spülung mit Wasser bewirkt hat, man in einem zweiten Schritt der Fixierung der aktiven Verbindung vorgeht.

3. Verfahren nach Anspruch 1, **dadurch gekennzeichnet, dass** man gleichzeitig mit der Reduktion der Disulfidbindungen des Keratins und der Fixierung der aktiven Verbindung vorgeht.

4. Verfahren nach einem der vorstehenden Ansprüche, **dadurch gekennzeichnet, dass** die Reduktion in einer Tiefe von etwa 4 bis 5 µm erfolgt.

5. Verfahren nach einem der vorstehenden Ansprüche, **dadurch gekennzeichnet, dass** die Reduktion realisiert wird, indem zwischen 0,1 und 5 Gew.% Cystein, bezogen auf alle Aminosäuren der Keratinfasern der Haare, erzeugt werden.

6. Verfahren nach Anspruch 5, **dadurch gekennzeichnet, dass** die Reduktion realisiert wird, indem man zwischen 0,1 und 2 Gew.% Cystein, bezogen auf alle Aminosäuren der Keratinfasern der Haare, erzeugt.

7. Verfahren nach einem vorstehenden Ansprüche, **dadurch gekennzeichnet, dass** das Reduktionsmittel ein Phosphin der allgemeinen Formel ist: 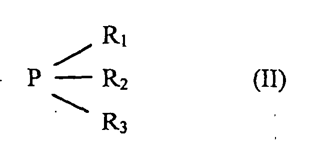
worin:
R₁, R₂ und R₃ gleich sind und darstellen:
(a) -(CH₂)ₙ-CH₃
(b) 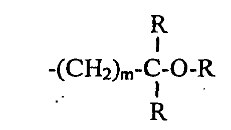
(c) -(CH₂)ₙ-COOR
(d) -(CH₂)ₙ-CONRR' und
(e) -(CH₂)ₙ-NRR'
n = 1 bis 3 ist,
m = 0 oder 1 bis 3 ist, und
R und R' gleich oder verschieden sind und ein Wasserstoffatom oder einen linearen oder verzweigten C₁-C₄-Alkylrest darstellen, und die Salze einer mineralischen oder organischen Säure der Verbindungen der Formel (II).

8. Verfahren nach Anspruch 7, **dadurch gekennzeichnet, dass** die Salze der Phosphine der Formel (II) ausgewählt sind unter den Säurechloriden, Säurebromiden, Sulfaten, Citraten, Oxalaten und Acetaten.

9. Verfahren nach einem der Ansprüche 7 bis 8, **dadurch gekennzeichnet, dass** das Phosphin ausgewählt ist unter Tris(2-carboxyethyl)phosphin und Tris(hydroxymethyl)phosphin.

10. Verfahren nach einem der Ansprüche 7 bis 9, **dadurch gekennzeichnet, dass** das Phosphin in einer Konzentration zwischen 10⁻³ M und 1 M vorliegt.

11. Verfahren nach einem der vorstehenden Ansprüche, **dadurch gekennzeichnet, dass** der pH-Wert der Zusammensetzung des Reduktionsmittels zwischen 3 und 9 liegt und vorzugsweise zwischen 4 und 7 liegt.

12. Verfahren nach einem der vorstehenden Ansprüche, **dadurch gekennzeichnet, dass** die Kontaktdauer der wässrigen Lösung des Reduktionsmittels mit den Keratinfasern zwischen etwa 30 Sekunden und einer Stunde beträgt und die Temperatur zwischen Umgebungstemperatur und einer Temperatur unterhalb von 60 °C liegt.

13. Verfahren nach einem der vorstehenden Ansprüche, **dadurch gekennzeichnet, dass** die aktive Verbindung ausgewählt ist unter den Farbstoffen, den UV-Filtern, den Glanzmitteln und hydrophoben Verbindungen, wobei die aktive Verbindung mindestens eine kernflüchtige Gruppe (Fluchtgruppe) trägt.

14. Verfahren nach einem der vorstehenden Ansprüche, **dadurch gekennzeichnet, dass** die aktive Verbindung in wässriger Lösung in einer Konzentration zwischen 10⁻³ % und 20 % verwendet wird, wobei die Lösung einen pH-Wert zwischen etwa 2 und 10 aufweist.

15. Verfahren nach einem der vorstehenden Ansprüche, **dadurch gekennzeichnet, dass** die Kontaktdauer der wässrigen Lösung der aktiven Verbindung im allgemeinen zwischen etwa einer Minute und einer Stunde beträgt und die Temperatur zwischen Umgebungstemperatur und einer Temperatur unterhalb von 60 °C liegt.

## Claims

1. Method for treating keratinous hair fibres to endow them with novel appropriate properties, **characterized in that** it comprises steps consisting in reducing the disulphide bonds of the hair keratin with a view to generating reactive sites only on the surface of said fibres to a depth of less than 10 µm, reducing of the disulphide bonds being realized using a reducing agent chosen from thiols neutralized using a polyquaternary ammonium hydroxide, phosphines and phosphites, hyperbranched polymers and dendrimers carrying terminal thiols functions, and in covalently fixing at least one active compound which is capable of endowing the keratinous hair fibres with novel appropriate properties on said reactive sites, said active compound comprising at least one reactive function which is capable of reacting with said reactive sites formed on the surface of the keratinous fibres.

2. Method according to Claim 1, **characterized in that** in a first step, the disulphide bonds of the keratin are reduced and **in that**, after optional rinsing with water, a second step is then carried out in which the active compound is fixed.

3. Method according to Claim 1, **characterized in that** reduction of the disulphide bonds of the keratin is carried out simultaneously with fixing of the active compound.

4. Method according to any one of the preceding claims, **characterized in that** reduction is carried out to a depth of about 4 to 5 µm.

5. Method according to any one of the preceding claims, **characterized in that** reduction is carried out to generate 0.1% to 5% by weight of cysteine with respect to the total amino acids of the keratinous hair fibres.

6. Method according to Claim 5, **characterized in that** reduction is carried out in order to generate 0.1% to 2% by weight of cysteine with respect to the total amino acids of the keratinous hair fibres.

7. Method according to any one of the preceding claims, **characterized in that** the reducing agent is a phosphine with general formula: 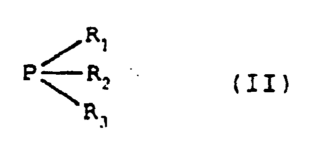
where:
R₁, R₂ and R₃, which are identical, represent:
(a) - (CH₂)ₙ-CH₃
(b) 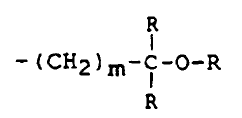
(c) -(CH₂)ₙ-COOR
(d) - (CH₂)ₙ-CONRR' and
(e) -(CH₂)ₙ-NRR'
n = 1 to 3
m = 0 or 1 to 3
R and R', which may be identical or different, represent a hydrogen atom or a linear or branched C₁-C₄ alkyl radical and salts of said compounds with formula (II) with a mineral or organic acid.

8. Method according to Claim 7, **characterized in that** the phosphine salts with formula (II) are selected from hydrochlorides, hydrobromides, sulphates, citrates, oxalates and acetates.

9. Method according to any one of Claims 7 to 8, **characterized in that** the phosphine is selected from tris(2-carboxyethyl)phosphine and tris(hydroxymethyl)phosphine.

10. Method according to any one of Claims 7 to 9, **characterized in that** the phosphine is present in a concentration in the range 10⁻³ M to 1 M.

11. Method according to any one of the preceding claims, **characterized in that** the pH of the reducing agent composition is in the range 3 to 9, preferably in the range 4 to 7.

12. Method according to any one of the preceding claims, **characterized in that** the contact time for the aqueous reducing agent solution with the keratinous fibres is in the range from about 30 seconds to 1 hour, the temperature being in the range from room temperature to a temperature of less than 60°C.

13. Method according to any one of the preceding claims, **characterized in that** the active compound is selected from colorants, sunscreens, shine agents and hydrophobic compounds, said active compound carrying at least one nucleofugic function.

14. Method according to any one of the preceding claims, **characterized in that** the active compound is used in an aqueous solution at a concentration in the range from about 10⁻³% to 20%, the pH of said solution being in the range from about 2 to 10.

15. Method according to any one of the preceding claims, **characterized in that** the contact time for the aqueous solution of active compound is generally in the range from about 1 minute to 1 hour, the temperature being in the range from room temperature to a temperature of less than 60°C.
